# EUROPEAN PATENT SPECIFICATION

(11) **EP 0 682 108 B1**
(45) Date of publication and mention of the grant of the patent: **10.12.1997**
(21) Application number: 95106554.9
(22) Date of filing: 02.05.1995
(51) Int. Cl.: C12N 7/00, A61K 39/245

(54) **Virus strain against pig pseudorabies and vaccines containing the said virus**
Virale Stämme gegen Pseudorabies vom Schwein und diese virale Stämme enthaltende Impfstoffe
Souche virale contre le pseudorabies du porc et vaccins contenant ledit virus

(30) Priority: 10.05.1994 IT MI940920
(43) Date of publication of application: 15.11.1995
(73) Proprietor: FATRO S.p.A., I-40064 Ozzano Emilia (Bologna) (IT)
(72) Inventor: Lomniczi, Béla, H-1124 Budapest (HU); Meliota, Francesco, I-40064 Ozzano Emilia (Bologna) (IT)
(74) Representative: Minoja, Fabrizio

(56) References cited:
- MAGYAR ALLATORVOSORK LAPJA, vol. 45, no. 8, 1990, pages 467-472; KÜKEDI, A. ET AL.: 'Molecular epidemiology of Aujeszky's disease virus'
- AM. J. VET. RES., vol. 51, no. 10, 1990, pages 1656-1662; L.M. HENDERSON ET AL.: 'In vivo and in vitro genetic recombination between conventional and gene-deleted vaccine strains of pseudorabies virus'
- JOURNAL OF VIROLOGY, vol. 61, no. 3, 1987, pages 796-801; B. LOMNICZI ET AL.: 'Genome location and identification of functions defective in the Bartha vaccine strain of pseudorabies virus'
- JOURNAL OF VIROLOGY, vol. 61, no. 12, 1987, pages 4030-4032; T.C. METTENLEITER ET AL.: 'Role of a structural glycoprotein of pseudorabies in virus vaccine'

## Description

### Field of the invention

The present invention refers to a new viral strain of Suid herpesvirus 1, to the process for the preparation of the new viral strain and to vaccines against pig pseudorabies or Aujeszky disease, prepared from the new viral strain.

Aujeszky disease or pseudorabies is caused by a virus that belongs to the Herpesviridae family, that affects most of all pigs. In animals for reproduction the pathogenic role of the virus takes place mostly at the level of genitals: In the boar it causes transient hypofertility, in the pregnant sow abortion, embryonal death and stillbirth. In the piglets is observed mainly the nervous variant with high death rate and rare respiratory forms, with advancing age the incidence of the respiratory form increases and that of the nervous form decreases. When respiratory lesions are present one can observe a decrease in weight gain.

The pig represents the primary host, with localisation in various organs, and represents also the only reservoir for the Aujeszky virus. Other domestic and wild animal species are naturally infected: cattle, sheep, dogs, cats and rodents, but represent a dead end from the epidemiological point of view, in that the infection has a lethal consequence.

### State of the art

The virus responsible for Aujeszky disease belongs to the Herpesviridae family, Alpha Herpesvirinae subfamily, and according to the official taxonomy is called Suid Herpesvirus 1. The complete virion presents a diameter of 180 nm and a DNA double helix.

The viral strains isolated cannot be differentiated by standard serologic techniques, and therefore belong to a unique serotype, whereas one can observe a notable diversity in virulence and pathogenicity between the different strains.

Recently an analysis of the viral genome has been performed using restriction enzymes to type the strains so that different strains have been identified, with epidemiological, pathogenetic and prophylactic advantages.

On the basis of this research the genes that code for the proteins responsible for replication, virulence and pathogenicity and those responsible for the immunogenicity and protection have been identified.

The vaccine strains in use at present have been attenuated either by classic methods or through genetic engineering methods.

The vaccine strains with classic methods are the strain Bartha K61 and the strain Tatarov MK-25.

The first is a strain selected for the formation of plaques of small size in cell culture, resulting attenuated and deprived of the gI glycoprotein, although it still produces the enzyme Thymidine kinase (TK).

The strain Tatarov MK-25 has been selected through treatment with the mutagenic chemical bromo-uridine (BUDR), resulting in an attenuated mutant negative for TK but not for gI.

The strains attenuated by genetic engineering combine to some extent the features of the above mentioned strains K61 and MK-25: in general they have been deleted of the genes encoding TK (Thymidine kinase) to reduce virulence and the glycoprotein gI to distinguish the serum response of the pigs inoculated with them from that induced by the wild type strains: typical examples are the strains 783 (EP 0141458) and Begonia.

### Description of the invention

A new strain has been found, and is the object of the present invention, which shows a strong attenuation of pathogenicity and a excellent immunogenicity useful for use in vaccines against Aujeszky disease.

The new vaccines are characterised therefore by the fact that they are derived from a new viral strain, called LomBart strain, that has a strong immunogenicity, is not pathogenic and in addition contains a genetic marker that permits its differentiation from other vaccine strains in use against Aujeszky disease.

The new strain LomBart, subject of the present invention has been deposited at the European Collection of Animal Cell Culture (ECACC) with accession number V94012710.

The new LomBart strain has been obtained by recombination in tissue culture between the Bartha K61 and a wild type strain carrying a recognisable genetic marker: the strain that is obtained contains the genetic marker, is equally attenuated and is more immunogenic that the initial Bartha K61.

The new vaccine derived from the LomBart strain presents clear advantages with respect to known vaccines and these are:
- a complete safety upon use,
- a vaccination efficacy due to its major immunogenicity,
- the possibility of differentiating it from other vaccine strains due to the presence of the genetic marker in the LomBart strain. This brings advantages in the disease eradication plan in that it permits verification at whatever moment if the vaccine strain, used for live vaccines, is involved in genetic recombination events "in vivo".

The new LomBart strain, in accordance with the invention, can be used for the preparation of live vaccines or inactivated in monovalent formulations or in vaccines containing other live or inactive elements, for use against Aujeszky disease and other swine diseases, in vaccines containing other virulent live strains, if desired after previous attenuation, or virulent inactive strains, for use against Aujeszky disease and other diseases of zootechnical interest.

The vaccine can be prepared and commercialised in a liophilised suspension or solution form for administration by injection or orally. The pharmaceutical forms contain a dose of LomBart strain and eccipients, adjuvants and preservatives. The efficacious dose of the LomBart strain ranges from 10² and 10⁶ and above in the attenuated vaccines, is equal to at least 10⁸ in inactivated vaccines. The dose usually employed in live vaccines is of 10⁵-10⁶/animal. The indicated administration can be parenteral for inactivated vaccines, parenteral, endonasal or intradermic for live vaccines.

### Description of the drawings

Figure 1 represents the distribution of the viral DNA fragments in an agarose gel after treatment with the enzyme Kpn1. The band C (LomBart strain) shows the presence of the F1+F2 marker and the fragment I' resulting from deletion of the gI gene. Band A relates to the reference virulent strain whereas band B refers to the Bartha K61 strain.

Figure 2 represents the physical map of the viral strain LomBart DNA: the distribution of the restriction sites of the enzyme Kpn1. The drawing shows the anomalous restriction site of fragment F, that results in two subfragments (F1+F2) that constitute the marker; shows in addition the position of the gene I deletion.

Figure 3 represents the nasal excretion of the strain of the invention in 6 pigs during a time interval between 1 and 12 days from administration of 10⁷ PFU of the viral strain.

Figures 4 and 5 represent the body growth curves of non vaccinated (Figure 4) and vaccinated pigs with the Bartha and LomBart strains (Figure 5).

### Detailed description of the invention

The subject of the present invention, the LomBart strain, has been obtained by recombination in tissue culture between the original Bartha K61 (A. Bartha (1961): Magyar Allatorvosok Lapja, 16, 42-45) and the natural ADV-53 strain, isolated in Hungary in 1982, carrying a recognisable genetic marker (B. Lomniszi, E. Nagy, A. Kuked, L. ZSak (1989)- In: Vaccination and control of Aujeszky disease- J.T. Van Oirschot ed. Kluwer Academic Publ., Dordrecht/Boston,London).

The LomBart strain thus obtained differs from the Bartha K61 strain in two different properties:
a) it contains within its DNA an easily recognisable marker thanks to an additional restriction site,
b) it forms bigger plaques in chicken embryo fibroblast cultures, characteristic correlated with a greater immunogenicity, verified also experimentally.

### Description of the genetic marker of the LomBart strain.

The LomBart strain has an additional restriction site within its DNA recognisable with the enzyme Kpn1. This enzyme is also the best to reveal the genome deletion.

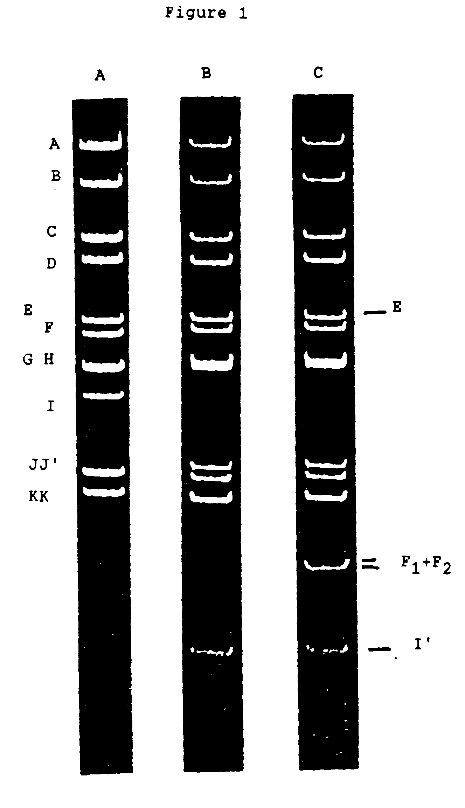

Figure 1 shows the distribution, after agarose gel electrophoresis, of the fragments of the strain Bartha K61, LomBart and of a wild type strain by comparison, obtained by treatment with the enzyme Kpn1. Whereas the virulent and Bartha K61 strains show the typical pattern of the prototype virus for pseudorabies, the fragment F of the LomBart strain does not appear at its usual position but runs "cut" in two fragments of more or less equal size between fragments K and I. This is because in this variant a restriction site sensitive to the restriction enzyme Kpn1 has appeared approximately in the middle of fragment F, thus resulting divided in the two subfragments F1 and F2.

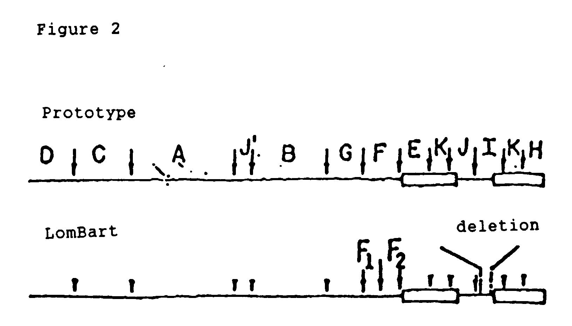

As foreseeable, the sum of the molecular weight of the two subfragments (F1=3.2:F2=3x10⁶ daltons) is equal to the molecular weight of fragment F (6.2x10⁶ daltons) (Figure 2).

The genetic marker described (identified by the sign Kpn F1+F2) is extremely rare in the viral strains isolated in Europe, and thus constitute an excellent characteristic to identify a viral strain.

### Construction of the LomBart strain

The source of the marker Kpn F1+F2 is constituted by the strain designated ADV-53, isolated in Hungary in 1982, that naturally possesses this characteristic.

To induce the marker Kpn F1+F2 in the vaccine strain K61 a natural recombination between the two strains was induced by co-culture in cell culture.

The cell line Pk15 has been infected at the same time with a culture containing about 10⁷ plaque forming units (P.F.U.) of the K61 strain and another culture containing about 10⁷ PFU of the ADV-53 strain. After absorbance for 1 hour at 37°C, the unbound viral particles were removed with repeated washes. The cell culture was incubated for 48 hours at 37°C, after which the supernatant was taken, centrifuged and the viral culture thus obtained titered using the plaque assay on chicken embryo fibroblast culture. Several dozens of plaques were isolated, the corresponding virus was cultured on Pk15 cells, purified by ultracentrifugation and the viral DNA was extracted and analysed by agarose gel electrophoresis after treatment with the restriction enzyme Kpn1.

The viral cultures thus cloned demonstrate the DNA marker F1+F2 and deletion of the Kpn1 fragment (Figure 1), and were further selected and cloned with the plaque method described above, and the new DNA analysed with the restriction enzymes Kpn1 and BamHI.

The analysis with the BamHI enzyme confirms that the virions whose DNA show a deletion of the fragment Kpn1, show also a deletion of the fragment Bam7, because these fragments partially correspond to each other in the viral genome. In this way the analysis with the restriction enzyme BamHI confirms the presence of the deletion in the DNA unique short region (Us) that is in turn responsible of the deletion of the gene coding for the gI glycoprotein.

Finally the virulence of the gI negative and F1+F2 positive viral mutants has been examined injecting at least 10⁵ PFU by the intracerebral administration route in 1 day old chicks; none of these mutants has shown pathogenicity.

One of these mutants has thus been selected, further cloned twice with the plaque method for all successive analysis and has been called "strain LomBart".

### Biological characteristics of the LomBart strain

### 1) Characteristics detectable "in vitro".

The Bartha K61 strain had been selected as an Aujeszky disease virus mutant that produces, when cultured on chicken embryo fibroblasts or on pig kidney cells, plaques of small size (K from Kicsi- small in Hungarian).

On the contrary, the LomBart strain forms slightly larger plaques than K61: 48 hours after infection of the cell cultures it is possible to note after coloration with neutral red plaques clearly visible of at least 1 millimeter in diameter. This plaque dimension and type are typical also of other vaccine strains (for example the strain 783 (EP 0141458) and DT-B (EP 0263207B1).

Despite the increase in plaque dimension with respect to the K61 strain, which reaches 1 millimeter diameter only after over 72 hours of culture, the margins of the plaques are as clean as those of plaques formed by fully virulent strains.

The minimal titer typical of LomBart strain cultures in stationary Pk15 cell cultures is of 3-5x10⁸ PFU/ml.

### 2) Deletion of the gI glycoprotein gene from the LomBart strain genome.

It has already been described above that examination by agarose gel electrophoresis of the LomBart strain DNA fragments generated by treatment with the restriction enzyme Kpn1 reveals that fragment I (corresponding to the Uₛ region of the genome) is smaller by about 3 millions daltons with respect to the virulent strain, so that it migrates much more rapidly in the agarose gel (the fragment indicated by I' in Figure 1).

This deletion is of the same dimension as that present in the Bartha K61 strain and results in deletion of the gI negative gene.

Deletion of the gI gene in the LomBart strain is further demonstrated by the fact that none of the piglets inoculated has ever shown seroconversion towards the gI protein after endonasal infection (Table 3). Even after repeated intramuscular injections for 4 months, the pigs do not show seroconversion against gI.

### 3) Innocuity of the LomBart strain.

### a) Absence of pathogenicity for 1 day old chicks.

It has been demonstrated several times that many Aujeszky disease attenuated viral strains are non virulent for newborn chicks (for example: Bartha K61 and strains Thymidine kinase negative), so that such demonstration is used routinely as screening for the strain pathogenicity. It is known that the virulent strains or some strains used in the past in live attenuated vaccines (the BUK strain) kill the chicks if inoculated intracerebrally at the dose of 10²-10³ PFU.

In the different assays carried out, cultures of LomBart strain containing 10⁵ PFU/µl have always been used. Groups of 5 one day old chicks have been inoculated by the intracerebral route (50µl) and maintained under observation together with a similar group inoculated with 50µl PBS.

All the inoculated chicks survived the infection without showing any clinical symptoms and grew normally, as well as the controls.

### b) Absence of pathogenicity in the sheep.

Sheep are extremely sensitive to the Aujeszky disease virus and for this reason they have always been used to verify the harmlessness of the vaccine strains, like Bartha K61.

The virulent Aujeszky disease strains kill sheep at doses below 10² PFU by the parenteral route.

**Example 1**: To evaluate the absence of virulence for sheep of the LomBart strain, 5 sheep have been injected intramuscularly at the neck with 5x10⁶ viral PFU, kept under observation for clinical symptoms and body temperature.

None of the sheep has shown symptoms, weight loss or fever for 2 weeks post experimental infection.

### c) Absence of pathogenicity in piglets.

The LomBart strain is absolutely inocuous for the pig.

**Example 2**: 5 seven week old piglets obtained from disease free breeding farms and absolutely deprived of antibodies have been infected by the endonasal route with 2x10⁶ PFU of LomBart strain. The body weight and temperature were measured daily for 14 days post infection and nasal samplings were performed to titer the excreted virus.

None of the piglets has shown clinical symptoms or fever and no decrease in the body weight gain index has occurred.

The virus has multiplied at the level of the nasal mucosa and has been excreted at values of about 10⁵ PFU/ml for 7-8 days: the mean excretion of the LomBart strain is slightly greater than that detectable with the Bartha K61 strain.

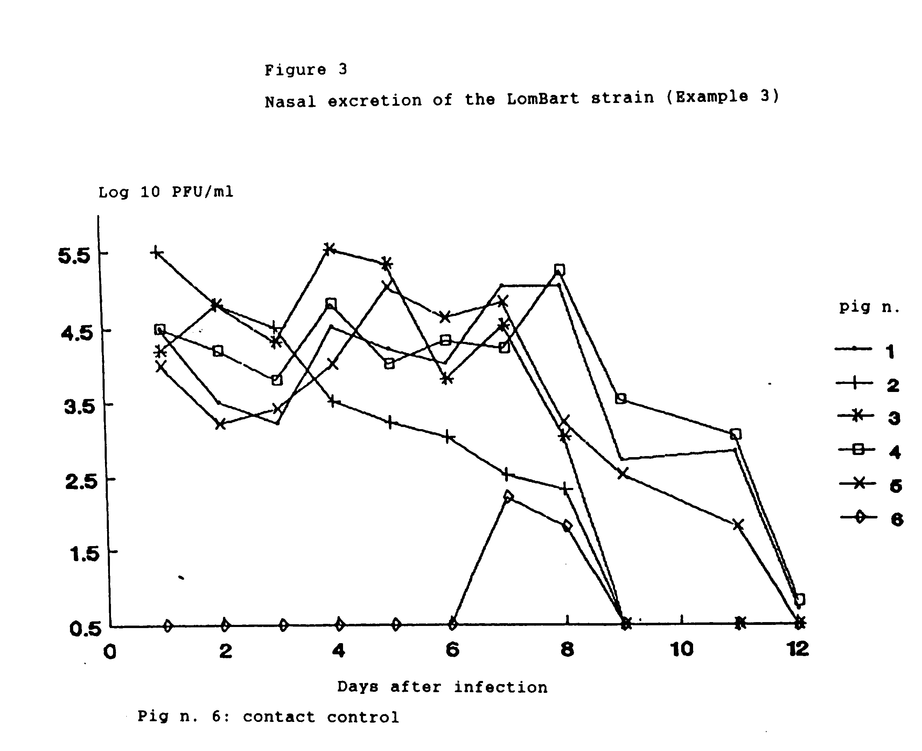

**Example 3**: Has been carried out exactly in the same manner, using four week old piglets, infected by the endonasal route with 10⁷ PFU of the LomBart strain. Also in this case the piglets remained absolutely free of clinical symptoms, grew normally and the level of virus excreted by the nasal route was of about 10⁵ PFU/ml (Figure 3 and tables 1 and 2).

In both assays the ELISA antibody titer reached by 14 days levels varying from 400 to 600, typical in piglets infected with vaccine strains.

**Table 1:**

| Weight of four week old piglets after endonasal infection with 10⁷ PFU of the LomBart strain | | | | | | | |
|---|---|---|---|---|---|---|---|
| Piglet N° | Days after infection | | | | | | |
| | **0** | **2** | **4** | **6** | **8** | **11** | **14** |
| **1** | **6,0** | **6,5** | **6,9** | **7,4** | **7,6** | **7,8** | **7,8** |
| **2** | **5,7** | **5,6** | **6,0** | **6,7** | **7,7** | **8,2** | **10,2** |
| **3** | **5,8** | **5,8** | **5,9** | **6,5** | **7,2** | **7,6** | **9,2** |
| **4** | **4,7** | **5,0** | **5,5** | **5,8** | **6,3** | **6,5** | **8,2** |
| **5** | **5,4** | **5,7** | **6,0** | **6,9** | **7,8** | **8,2** | **9,7** |
| **6*** | **6,9** | **6,9** | **7,8** | **8,5** | **9,3** | **9,6** | **11,7** |

| | | | | | | | |
|---|---|---|---|---|---|---|---|
| *: control piglet in contact with the other subjects | | | | | | | |

**Table 2:**

| seroconversion of 4 week old piglets after endonasal infection with 10⁷ PFU of the LomBart strain. | | | | | |
|---|---|---|---|---|---|
| Piglet N. | Days after infection | | | | |
| | **0** | **12** | | **22** | |
| | **AY-ELISA** | **AY-ELISA** | **gI** | **AY-ELISA** | **gI test** |
| **1** | **< 50** | **1:200** | **--** | **1:400** | **--** |
| **2** | **< 50** | **200** | **--** | **400** | **--** |
| **3** | **< 50** | **200** | **--** | **800** | **--** |
| **4** | **< 50** | **400** | **--** | **1600** | **--** |
| **5** | **< 50** | **400** | **--** | **800** | **--** |
| **6** | **< 50** | **1600** | **--** | **1600** | **--** |

### 4) Immunogenicity and protective efficacy against wild type infections.

### a) Immunogenicity

**Example 4**: week old piglets were immunised with a live attenuated vaccine made of LomBart strain cultures, at decreasing doses from 10⁶ to 10¹ PFU; even 30 10 PFU were shown able to induce an antibody response in the vaccinated animals. The ELISA titer of the antibodies varied from 100 to 800 without correlation with the vaccine dose. When the piglets underwent a recall vaccine 1 month after the first injection, it was possible to observe an increase in the secondary antibodies characterised by an ELISA antibody level varying from 800 to 3200 (Table 3).

Table 3: Immunogenicity of the LomBart strain: 10 week old piglets vaccinated twice (with a 30 day interval) with decreasing doses of virus.

ELISA titers expressed as Log2⁺_{_} standard deviation-groups of 5 pigs

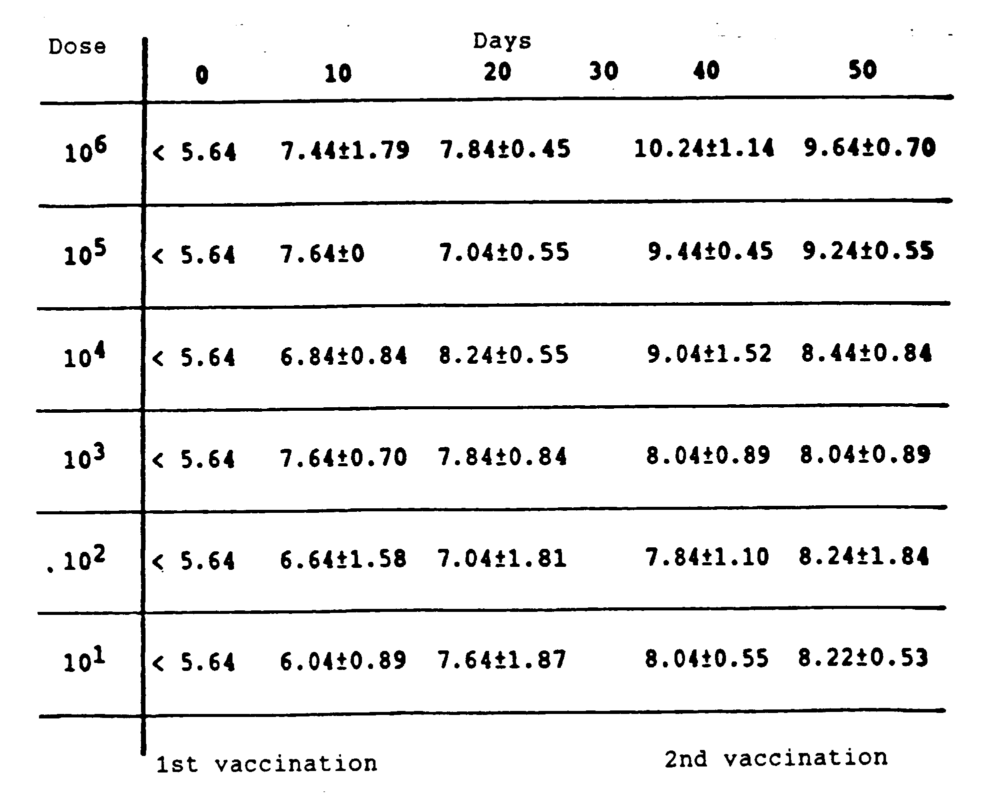

### b) Protection by the vaccine

**Example 5**: In experiments carried out according to the classic protocol for the evaluation of vaccines against Aujeszky disease, groups of 5 ten week old piglets were vaccined with a vaccine containing 10⁵ PFU of the LomBart strain; after 4 weeks, the vaccinated pigs and the control unvaccinated group (5 subjects per group) were infected by the endonasal route with 10⁴ PFU of virulent virus (the Rice strain).

After the experimental infection the control pigs manifested grave Aujeszky disease symptoms: an growth arrest for 10-12 days, fever >41°C, respiratory disorders for many days, respiratory symptoms for over a week, nervous symptoms in 1-2 subjects in each group with consequent death of the pigs involved.

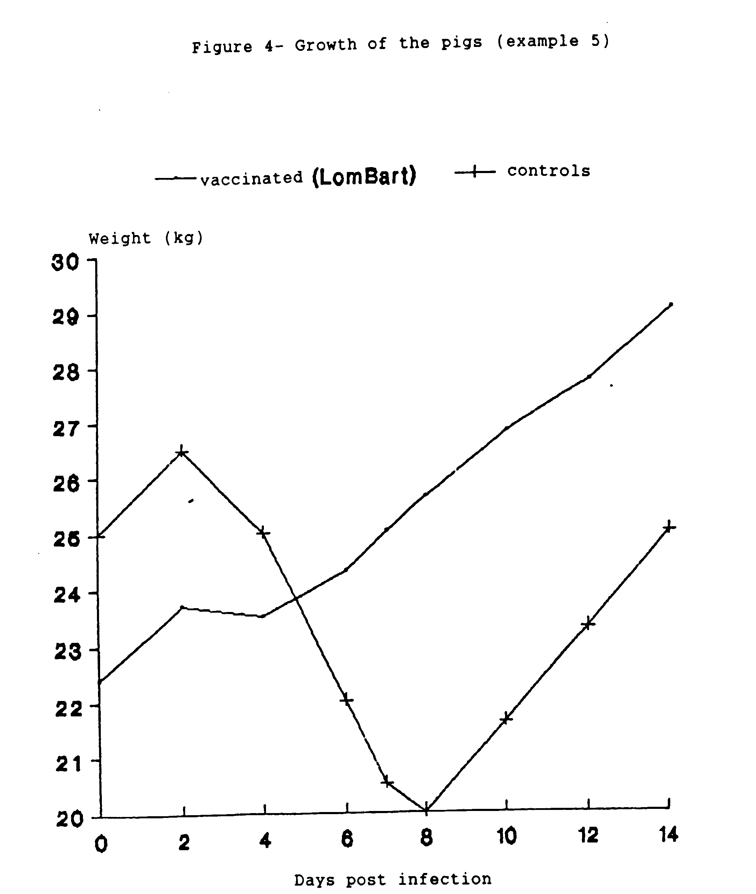

On the contrary, in the vaccinated groups the clinical symptoms were greatly reduced: the growth arrest was limited to 1-3 days, the fever was low (<41°C) in the absence of any respiratory and nervous symptomatology and death (Fig. 4).

The nasal excretion of wild type virus is only slightly reduced following a single vaccination: Excretion lasts for about 10 days (compared to 15 in the non-vaccinated surviving pigs) and also the total amount of eliminated virus is reduced.

In other experiments the pigs were vaccinated twice at a distance of 4 weeks, then infected after a further 4 weeks: in this case, in addition obviously to the total protection from the clinical symptomatology and from fever, a further decrease in wild type virus nasal excretion was seen: the duration of the elimination was below 8 days and the virus titers were less by 1-2 logarithms compared to controls.

The level of protection offered by live attenuated vaccines based on the LomBart strain is therefore comparable to that observed in the same experimental conditions, with vaccines based on thymidine kinase negative strains.

### c) Increase in the vaccine protection with respect to the Bartha K61 strain.

**Example 6**: The greater immunogenicity of the LomBart strain compared with the Bartha K61 strain can be indirectly inferred on the basis of its characteristic of forming plaques with a larger diameter in chicken embryo fibroblasts.

To experimentally check that the LomBart strain is more protective, it has been tested in comparison with the Bartha strain in live vaccines containing low virus titers, to amplify the immunogenicity differences detectable.

4 groups of 6 ten week old piglets were vaccinated with vaccines based on the LomBart strain or on the K61 strain, two groups at the dose of 10 PFU/subject (1 LomBart + 1 K61) , the other at the dose of 10²/PFU/subject. After 4 weeks, the vaccinated pigs were infected by the endonasal route with 10⁴ PFU of virulent virus.

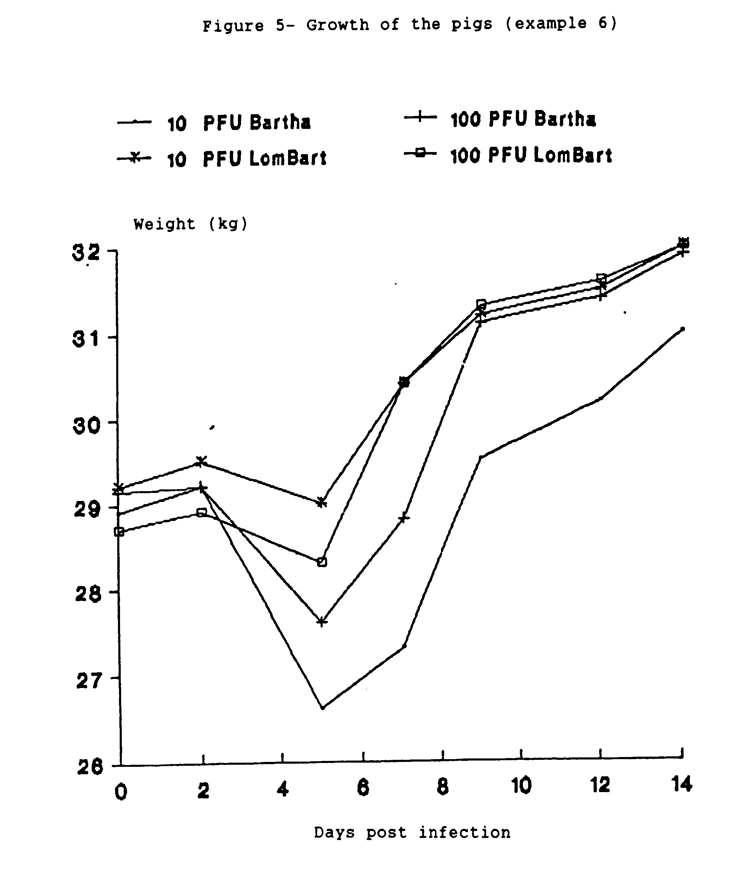

None of the pigs vaccinated with products based on the LomBart strain has shown clinical symptoms. The pigs vaccinated with the K61 strain have shown grave respiratory symptoms and a certain delay in growth, all of which in a more accentuated manner in the subjects vaccinated with lower doses (Figure 5 and Table 4).

**Table 4:**

| N. of pigs with grave respiratory symptoms following experimental infection (groups of 6 pigs) Pigs vaccinated with the LomBart or Bartha strains | | | | | | | | | |
|---|---|---|---|---|---|---|---|---|---|
| Vaccine dose | | Day post infection | | | | | | | |
| | | **3** | **4** | **5** | **6** | **7** | **8** | **9** | **10** |
| **LOMBART** | **10**^{**1**} | **-** | **-** | **-** | **-** | **-** | **-** | **-** | **-** |
| | **10**^{**2**} | **-** | **-** | **-** | **-** | **-** | **-** | **-** | **-** |
| | | | | | | | | | |
| **BARTHA** | **10**^{**1**} | **-** | **-** | **4** | **4** | **6** | **2** | **-** | **-** |
| | **10**^{**2**} | **-** | **-** | **3** | **3** | **5** | **2** | **-** | **-** |

### Preparation of vaccines from the lombart strain.

**Example 7**: Preparation of a live attenuated vaccine against Aujeszky disease from the LomBart strain.

### A. Culture of the virus.

Cells of the continuous cell line Pkl5, cultured in monolayers, are infected with the LomBart strain. After 24 hours of incubation at 37°C the cytopathic effect caused by the virus is already evident. After a further 48 hours of growth, the viral culture is collected, the cells and cellular debris removed by centrifugation in a cooled centrifuge, the supernatant mixed with an equal volume of the stabilizer SPGA and frozen at -70°C.

The titer of the viral content is made on Pk15 cells with an aliquot of the viral culture.

Once the result of the titer has been obtained, the viral culture is rapidly thawed and distributed in vials for lyophilisation, after having carried out a dilution in order to have a titer, at the and of the lyophilisation, not below 10⁵TCID₅₀/dose of vaccine.

The vaccine is then lyophilised and closed under vacuum at the end of the process.

**Example 8**: Preparation of an inactive vaccine against Aujeszky disease from the LomBart strain.

Suspension cultures of the continuous cell line BHK-21 are infected with the LomBart strain; after 40-48 hours of growth the cells and cellular debris are removed by centrifugation and/or filtration, a sample is removed from the liquid containing the virus for virus titering.

This culture is treated with Binary Ethylenimine for 12 hours at 37°C to inactivate the virus. At the end of the process of inactivation, an aliquot is removed to control for the viral inactivation whereas the antigen is kept at 4°C.

At the end of the titering, the antigen, at a titer of at least 10⁹TCID₅₀/dose calculated on the non inactivated culture, is adsorbed to aluminium hydroxide sterilised in an autoclave, to produce the inactive virus adsorbed to an aluminium hydroxide gel.

Alternatively, the antigen at a titer of at least 10⁸TCID₅₀/dose can be additionated with tensioactive compounds (for example mannitol monooleate, sorbitan monooleate, tween 80, Span 80) and with mineral or vegetable oils, or with a mixture of them, and then emulsionated with a homogeniser (of the Ultra Turrax, Silverson or other type), to make an inactive emulsionated vaccine.

## Claims

1. Pseudorabies virus derived from the recombination of the Bartha K61 strain with a strain carrying the genetic marker Kpn F1+F2.

2. Virus according to claim 1 deposited at the European Collection of Animal Cell Cultures under the n. V94012710.

3. Live or inactivated vaccines against pig pseudorabies that include a virus of the claims 1 or 2.

## Patentansprüche

1. Pseudorabies-Virus, stammend von der Rekombination des Bartha K61-Stammes mit einem Stamm, der den genetischen Marker Kpn F1+F2 trägt.

2. Virus nach Anspruch 1, hinterlegt bei der European Collection of Animal Cell Cultures unter der Nummer V94012710.

3. Lebende oder inaktivierte Vaccinen gegen Schweine-Pseudorabies, die ein Virus nach Anspruch 1 oder 2 umfassen.

## Revendications

1. Virus de la pseudo-rage dérivé de la recombinaison de la souche Bartha K61 avec une souche portant le marqueur génétique Kpn F1+F2.

2. Virus selon la revendication 1 déposé auprès de la Collection Européenne des Cultures Cellulaires Animales sous le numéro V94012710.

3. Vaccins actifs ou inactivés contre la pseudo-rage du porc qui comprennent un virus selon les revendications 1 ou 2.
